# EUROPEAN PATENT APPLICATION

(11) **EP 3 404 092 A1**
(43) Date of publication of application: **21.11.2018**
(21) Application number: 17171571.7
(22) Date of filing: 17.05.2017
(51) Int. Cl.: C12M 1/32, B04B 7/02, C12M 1/00, C12M 1/26

(54) **METHOD AND APPARATUS FOR CENTRIFUGATION-BASED ACCUMULATION AND COLLECTION OF CELL CULTURES**

(71) Applicant: Johann Wolfgang Goethe-Universität, 60323 Frankfurt am Main (DE)
(72) Inventor: Hötte, Katharina, 65929 Frankfurt am Main (DE); Pampaloni, Francesco, 65929 Frankfurt (DE); Stelzer, Ernst H. K., 74909 Meckesheim (DE)
(74) Representative: Lucke, Andreas

(57) **Abstract**

A cover lid collector (10) for a sample carrier (40) for cell cultivation, wherein the cover lid collector (10) comprises a connecting portion (12) for engaging with the sample carrier (40) for closing the same, wherein the connecting portion (12) defines a first plane (11). The cover lid collector (10) further comprises a cavity (14), which when the cover lid collector is engaged with the sample carrier (40), has a cross-section that decreases in a direction perpendicular to the first plane (11) and pointing away from said sample carrier (40) between a near end (16) of the cavity (14) and a far end (18) of the cavity (14), wherein the near end (16) is closer to the sample carrier (40) than the far end (18).

## Description

### FIELD OF THE INVENTION

The present invention is in the field of cell cultivation. In particular, the present invention relates to a method and an apparatus for accumulating and collecting biological samples comprised in a liquid medium, in particular a culture medium, contained in a sample carrier for cell cultivation.

### BACKGROUND OF THE INVENTION

A major goal of life sciences research is the pursuit of experiments in conditions as close as possible to naturally occurring conditions. For this purpose, cell cultures are employed, which are artificially-created environments in which cells are allowed to grow and to biologically interact. Contrary to traditionally used two-dimensional monolayer cell cultures, like for example those supported by a petri dish, three-dimensional cell cultures allow cells to grow and interact in all spatial directions, as would be the case in naturally occurring in vivo conditions. Therefore, three-dimensional cell culture systems have become increasingly interesting in areas such as drug discovery and tissue engineering.

However, the handling of three-dimensional cell cultures for experimental purposes has not yet achieved the degree of easiness and efficiency exhibited by well-established two-dimensional cell cultivation techniques that would allow for the translation of well proven experimental protocols of two-dimensional cell cultures to three-dimensional cell cultures. This is of particular concern, since for producing statistically reliable experimental results, a large number of identical samples has to be handled.

For this purpose, sample carriers, like for instance culturing dishes or microtiter plates, are typically employed in laboratory setups. Such sample carriers can contain a large number of samples, between a few and several thousands. For example, in the case of a microtiter plate, each of a large number of wells of the microtiter plate may be used as a test tube for receiving a liquid sample to be tested. A "sample" is understood herein as a test quantity of a biological sample comprised in a liquid medium. The distinction between "sample" and "biological sample" is hence intentional and meaningful in the context of this application, inasmuch as a sample is meant to comprise a biological sample like for example spheroid or organic cultures contained in a liquid, such as culture medium. In an alternative example, the samples may correspond to hanging drops that are attached to a cell culturing dish or to a hanging drop plate after having been obtained by means of the hanging drop technique. In any case, the biological samples are typically arranged in a sample carrier in a rectangular array.

Traditional techniques for handling samples contained in a sample carrier include for example pipetting or aspirating the samples at each of the wells individually. However, these techniques are far too time-consuming and too exposed to a high failure rate to produce reliable experimental results. Further, the aforesaid techniques have the additional drawback of an increased exposure of the samples to environmental conditions that may affect the results of the experiment in an unwanted way.

Alternatively, a liquid handling robot or pipetting robot may be employed for handling samples contained in a sample carrier. However, pipetting robots are expensive pieces of equipment that are not always available, and even if available require a certain amount of programming to adapt to the experimental setup to be used.

In view of this, there is room for technical improvements in the handling of three-dimensional cell cultures for experimental purposes.

### SUMMARY OF THE INVENTION

The problem underlying the invention is to provide an apparatus and a method that allow handling a large number of samples in a simple and efficient way with reduced exposure to environmental conditions in order to increase the feasibility and reliability of experimental results obtained by using three-dimensional cell cultures. This problem is solved by a cover lid collector according to claim 1 and by a method according to claim 11. Preferable embodiments of the invention are described in the dependent claims.

The invention refers to a cover lid collector for a sample carrier for cell cultivation. A sample carrier refers herein to a conventional experimental device configured for containing a sample or a plurality of samples, such that a large number of samples may be handled under the same environmental conditions. The sample carrier may for example be a microtiter plate, also known as multi-well plate, comprising a large number of wells, wherein each well may be used as a test tube for receiving a biological sample to be tested. The sample carrier may also comprise a so-called hanging drop plate, a cell-culturing dish, or any other device suitable for containing biological samples obtained by means of the hanging drop technique. Sample carriers are broadly used for experimental purposes in laboratories and are typically manufactured according to standardised shapes and dimensions.

The cover lid collector comprises a connecting portion for engaging with the sample carrier for closing the same, wherein the connecting portion defines a first plane. The shape, size and dimensions of the connecting portion of the cover lid collector may be tailored to a particular type or model of sample carrier such that the cover lid collector and the sample carrier may be tightly attached to one another by engaging the connecting portion with the sample carrier. The "first plane" refers herein to a geometrical plane defined by the connecting portion, which according to preferred embodiments of the invention may be oriented parallel to an extension plane of the sample carrier.

The cover lid collector further comprises a cavity, which, when the cover lid collector is engaged with the sample carrier, has a cross-section that decreases in a direction perpendicular to the first plane and pointing away from said sample carrier between a near end of the cavity and a far end of the cavity. The near end is closer to the multi-well plate than the far end. The cavity hence extends between the far end and the near end and the cross-section of the cavity, which is understood herein as the area of a plane parallel to the first plane defined by the connecting portion that is intersected by the cavity, decreases in the direction normal to the first plane from the near end towards the far end. The cavity is then in fluid communication with at least some of the samples comprised in the sample carrier. In some embodiments of the invention, the cross-section of the cavity is preferably maximal at the near end and minimal at the far end. The near end of the cavity may in some embodiments of the invention totally or partly coincide with or at least be comprised in the first plane defined by the connecting portion.

The cover lid collector of the invention can be used for handling samples, for example samples contained in one or more of the wells of a multi-well plate or samples contained as hanging drops in a culture dish, in order to collect and extract biological samples, like for example spheroid or organoid cultures, comprised in a liquid medium, like for instance a culture medium, by means of centrifugation. For this purpose, the cover lid collector may be used to close a sample carrier containing the samples by engaging the sample carrier and the connecting portion of the cover lid collector such that the cover lid collector and the sample carrier are tightly attached to each other via the connecting portion and the cavity forms a hollow space that fluidly connects the parts of the sample carrier that are configured for containing, receiving or supporting the samples. This means that the samples can potentially flow from the sample carrier into the cavity of the cover lid collector when the cover lid collector is engaged with the sample carrier.

The sample carrier covered with the cover lid collector may then be subjected to centrifugation, for example by means of a centrifuging device or centrifuge, in such a way that centrifugal forces arise in the direction normal to the first plane pointing and away from the sample carrier, i.e. pointing towards the far end of the cavity, such that the biological samples accumulate at the far end of the cavity. For this purpose, the cover lid collector of the invention may be adapted to the physical and mechanical properties of the centrifuge, such that the lid collector can be easily secured to the centrifuge. For instance, if the centrifuge comprises planar surfaces configured for receiving sample carriers, like e.g. swing-out plates, the cover lid collector may comprise a planar surface opposite to the first plane defined by the connecting portion and parallel thereto, i.e. parallel to the first plane, such that the cavity is disposed between said planar surface and the first plane. The cover lid collector engaged with the sample carrier may then be placed in the centrifuge resting on said planar surface. Note, however, that any other structural and geometrical configuration of the cover lid collector that makes it suitable for being used in combination with a given model or type of centrifuge is also possible.

The centrifugation results in fast collection of samples and a spatial redistribution of the components of the samples, i.e. of the liquid medium and the biological samples, depending on their constituent particle sizes and densities according to the principle of differential centrifugation. As a result of centrifugation, the biological samples, which are larger and denser than the constituent particles of the liquid medium, concentrate at the far end of the cavity forming a first phase separated from a second phase formed by the liquid medium, which concentrates further away from the far end than the biological samples.

Thus, the cover lid collector of the invention allows collecting biological samples comprised in samples contained in a sample carrier without requiring individual collection of each single sample contained in the sample carrier, thereby increasing the simplicity and the rapidness with which the biological samples may be extracted from the sample carrier, e.g. after incubation or drop formation therein for their analysis or further processing for experimental purposes. A large number of samples contained in a sample carrier may be processed in a single centrifugation step by means of the invention. This allows increasing the throughput of the experimental setups used for obtaining experimental results relying on three-dimensional cell cultures with respect to existing techniques.

Further, the invention allows collecting said biological samples in a way that minimises the contact of the biological samples with the ambient atmosphere or external agents or devices that would otherwise be required for the collection and for the extraction of the biological samples and hence contributes to an improved degree of sterilisation of cell cultivation, thereby reducing the risk of contamination that may influence the result of cell cultivation experiments.

In addition, the simple structural and material constitution of the cover lid collector of the invention, which may for example be made of a plastic material such as polystyrene, PET, POM, FEP, PFTE, silicon, and further bio-compatible materials, including plastic, elastomers, rubbers, silicates and metal alloys, allows for its manufacture in an efficient and cost saving manner, which makes the cover lid collector of the invention suitable for mass production and affordable single use. In addition, the cover lid collector of the invention may be reused. Thus, the invention provides a solution for improving the feasibility of biological assays based on three-dimensional cell cultures, which is also attractive from a cost perspective.

In a preferred embodiment of the invention, the cross-section of the cavity decreases monotonically in said direction perpendicular to the first plane and pointing away from the sample carrier between the near end of the cavity and the far end of the cavity. Thus, the cross-section of the cavity is maximal at the near end and minimal at the far end and the cross-section of the cavity steadily decreases in the direction from the near end towards the far end.

According to a preferred embodiment of the invention, the cavity is formed by a plurality of planar walls that extend between the near end and the far end, such that the cross-section of the cavity has a polygonal shape. The planar walls preferably have a smooth surface that prevents any attachment of the biological samples, which could otherwise impede their concentration at the far end of the cavity during centrifugation or harm the integrity of the samples.

In a preferred embodiment of the invention, at least one of the planar walls has a polygonal shape, preferably a triangular shape. However, the planar walls may as well have other polygonal shapes, be it a regular polygonal shape, like a rectangular shape, a trapezoidal shape, a square shape and the like, or an irregular polygonal shape.

According to preferred embodiments of the invention, one or more of the planar walls has a slope angle that is different to the slope angle of another one of the planar walls, wherein the slope angle is an angle defined between the surface of the wall and the first plane defined by the connecting portion of the cover lid collector. The slope angle hence measures the inclination of each of the planar walls with respect to the first plane, wherein, as a technically unfeasible example used for the purposes of understanding, a planar wall perpendicular to the first plane would be characterised by a slope angle of 90°, whereas a planar wall coincident with the first plane would be characterised by a slope angle of 0°. Note that, when at least one of the planar walls has a slope angle that is different to the slope angle of another wall, the cavity may have a regular or an irregular polyhedral shape depending, e.g. on the shape and orientation of the far end of the cavity.

According to a preferred embodiment of the invention, the planar walls have a common vertex that forms the far end of the cavity. The vertex forms a peak of the cavity that may render the collection of the biological samples of the samples contained in the sample carrier easier once they have concentrated around the far end of the cavity after centrifugation. For instance, the biological samples may easily be collected by means of an aspirator by concentrating the aspirating action thereof at said vertex.

According to preferred embodiments of the invention, the cavity is formed by four triangular shaped walls, wherein said four walls are pairwise adjacent and opposed, such that the cavity has a pyramidal shape, a symmetric quasipyramidal shape or an asymmetric quasipyramidal shape. For example, two of said walls may be opposed to each other and have a first slope angle while the other two of said walls may be opposed to each other and have respectively a second and a third slope angle, such that the cavity has a quasipyramidal shape. In this case, the near end of the cavity defining the exterior opening profile thereof may have a rectangular shape that may coincide with the first plane defined by the connecting portion of the cover lid collector or at least be parallel thereto. The vertex of the quasipyramid at which the four walls meet may or may not be centered with respect to the plane that constitutes the base of the quasipyramid.

Alternatively, each of said four walls may have a slope angle different to the slope angle of the three other walls, such that the cavity has an asymmetric quasipyramidal shape. The near end of the cavity defining the exterior opening profile thereof may still have a rectangular shape that may coincide with the first plane defined by the connecting portion of the cover lid collector or at least be parallel thereto.

In a preferred embodiment of the invention, the cover lid collector further comprises a drain opening that fluidly connects the far end of the cavity to the exterior of the cover lid collector. This allows easily extracting the biological samples after centrifugation of the samples through the drain opening. In embodiments in which the cover lid collector comprises a planar surface opposite to the first plane defined by the connecting portion and parallel thereto, the opening may be configured for connecting the far end of the cavity to the exterior of the cover lid collector through said planar surface. Preferably, the opening is then aligned with the plane of the planar surface without protruding from the planar surface.

According to a preferred embodiment of the invention, the cover lid collector further comprises a sealing cap configured for tightly closing the drain opening in order to avoid that the samples or parts thereof uncontrollably pour out of the experimental setting formed by the sample carrier and the cover lid collector during centrifugation or manipulation.

According to preferred embodiments of the invention, the cover lid collector further comprises a sealing element configured for being arranged around the cover lid collector and around the sample carrier so as to provide a tight sealed connection between the cover lid collector and the sample carrier when the cover lid collector is engaged with the sample carrier. The sealing element may be used to cover the connecting portion of the cover lid collector when the connecting portion is engaged with the sample carrier in order to provide a tight connection thereof. The sealing element may have a band-like-shape and may be of any suitable sealing material, preferably of an elastic material like e.g. silicon or rubber, and it may have a perimeter exceeding the outside perimeter of the connecting portion of the cover lid collector by a few millimetres, for instance by 1 to 5 mm. This allows providing a tight connection between the cover lid collector and the sample carrier even when the dimensions of the connecting portion of the cover lid collector do not exactly match those of the sample carrier. This may allow overcoming technical problems due to manufacturing tolerance of the sample carriers and of the cover lid collector as well as extending the range of applicability of the cover lid collector of the invention to sample carriers other than the particular type or model of sample carrier for which the cover lid collector has been designed.

In a preferred embodiment of the invention, the cavity and/or at least one of the planar walls thereof may be totally or partly coated with a magnetic material. This may help collecting biological samples that react to magnetic forces, like e.g. biological samples labelled with magnetic antibodies against surface proteins.

According to a preferred embodiment of the invention, the cavity and/or at least one of the planar walls thereof may be partly or totally coated with an antiadhesive coating. This may prevent biological samples comprised in the samples contained in the sample carrier from remaining attached to the surfaces of the cavity, i.e. to the walls, and to instead move towards the far end of the cavity during centrifugation to concentrate there. Consequently, the efficiency of the handling of biological samples contained in a sample carrier with the cover lid collector of the invention can be improved. The antiadhesive coating, which may be formed by any suitable technique, like e.g. coating or etching, may for example comprise passivation/functionalization of polystyrene, PET, POM, perfluor-polymers such as polytetrafluorethylene (PTFE), silicates, as well as elastomers such as PDMS and further silicon rubbers, which may be obtained by surface chemistry procedures.

According to another preferred embodiment of the invention, the cavity and/or at least one of the planar walls thereof may be partly or totally coated with an adhesive coating. The adhesive coating may for example comprise immobilized antibodies, a histidine-tags extracellular matrix or the like. This may allow extracting specific cell types comprised in the biological samples by selecting an adhesive coating such that specific cell types to be extracted do not adhere to the adhesive coating while other cell types that should not be extracted do adhere to the adhesive coating and are hence not collected at the far end of the cavity.

In other preferred embodiments of the invention, the cavity and/or at least one of the planar walls thereof may be partly or totally coated with an adhesive coating. The adhesive coating may for example be configured for selectively preventing biological samples to move towards the far end of the cavity during centrifugation to concentrate there according to their size or type. This may allow selectively separating biological samples of a given type at the far end of the cavity from other types of biological samples by preventing biological samples of said given type from concentrating at the far end of the cavity during centrifugation.

In a preferred embodiment of the invention, the cover lid collector further comprises a removable separating foil covering the near end of the cavity. The removable separating foil may be configured for preventing uncontrolled leakage of the samples contained in the sample carrier when manipulating the same for example for engaging with the cover lid collector. The removable separating foil may for example cover each one of the wells of a multi-well plate so as to ensure that the biological samples remain in the respective wells and do not spill out or mix in an uncontrolled way before this is actually desired. The removable separating foil may further be configured for being removed from the outside of the cover lid collector when the cover lid collector is engaged with the sample carrier. Further, the removable separating foil may be configured for disintegrating when entering in contact with the biological samples or to be disrupted by centrifugal forces upon centrifugation, such as to let the biological samples flow freely within the cavity during centrifugation.

According to preferred embodiments of the invention, the cover lid collector is fully or partially made of a transparent material, such that the interior of the cavity can be visually inspected from the outside of the cover lid collector. This may allow observing the formation of phases in the samples from the outside of the cover lid collector during and/or after centrifugation.

In preferred embodiments of the invention, the cover lid collector comprises more than one cavity, wherein each cavity may have properties according to any of the embodiments described above. For example, the cover lid collector may comprise a first and a second cavity, wherein, when the cover lid collector is engaged with the sample carrier, a first part of the sample carrier is in communication with the first cavity, wherein said first part of the sample carrier may contain a first part of the samples contained in the sample carrier, whereas a remaining second part of the sample carrier is in communication with the second cavity, wherein said second part of the sample carrier may contain the remaining second part of the samples contained in the sample carrier, namely those samples not contained in the first part of the sample carrier.

When the sample carrier and the cover lid collector are engaged together and subjected to centrifugation to separate the biological samples from the liquid medium, biological samples comprised in the samples contained in the first part of the sample carrier may be collected at the far end of the first cavity and biological samples comprised in the samples contained in the second part of the sample carrier may be collected at the far end of the second cavity. Note that although an embodiment of the cover lid collector comprising two cavities is described herein for illustrative purposes, the number of cavities is not restricted thereto. Alternative embodiments having a higher number of cavities are also possible. Also note that the first and second cavities, and/or any other cavities of the cover lid collector, need not necessarily be identical and may in particular conform to different ones of the embodiments described above.

A further aspect of the invention is related to a method of separating biological samples comprised in a liquid medium, in particular a culture medium, the liquid medium being contained in a sample carrier for cell cultivation, using a cover lid collector according to any of the embodiments described above, wherein the method comprises the steps of:
- engaging the cover lid collector with the sample carrier, such that the cover lid collector is tightly connected to the sample carrier;
- disposing cover lid collector engaged with the sample carrier in a centrifuge, such that the cover lid collector is positioned over the sample carrier;
- centrifuging the cover lid collector engaged with the sample carrier such as to subject the liquid medium and the biological samples to a centrifugal force in said direction, such that the liquid medium forms a first phase and the biological samples form a second phase, wherein the first phase and the second phase are separated from each other and wherein the second phase is closer to the far end of the cavity than the first phase; and
- extracting the second phase from the cover lid collector.

The step of extracting the second phase, i.e. the biological samples, from the cover lid collector may comprise, e.g. aspirating or pipetting, for instance by means of an aspirator or a syringe.

According to a preferred embodiment of the invention, the cover lid collector further comprises a drain opening that fluidly connects the far end of the cavity to the exterior of the cover lid collector, wherein extracting comprises extracting the second phase by draining off the biological samples through the drain opening.

In a preferred embodiment of the invention, centrifuging comprises centrifuging at different centrifugation speeds. The choice of the centrifugation speeds may be made in consideration of the physical characteristics of the samples and of their components, for example taking into account the biological samples to be collected during centrifugation at the far end of the cavity.

Changes in the centrifugation speed may be made at any stage during the execution of the method. In particular, the method steps may be repeated before and after changing the centrifugation speed in order to allow different kinds of biological samples present in the samples to be subsequently collected by means of centrifugation and extracted from the cover lid collector for further analysis or experimentation. Further, the centrifugation speed may be changed several times before the extracting step is carried out such that several phases are subsequently formed, e.g. by biological samples of different kinds, which may then be subsequently extracted from the cover lid collector.

For example, a sample carrier may contain biological samples comprising biological samples of a first type and biological samples of a second type contained in a buffer medium. The sample carrier may then be engaged with the cover lid collector and subjected to centrifugation at a first centrifugation speed until the biological samples of the first type concentrate at the first end of the cavity forming a first phase differentiated from the rest of the sample. The biological samples of the first type may then be extracted from the cover lid collector. Then, the sample carrier engaged in the cover lid collector may be subjected to centrifugation at a second centrifugation speed until the biological samples of the second type, which are still present in the sample, concentrate at the first end of the cavity forming a second phase differentiated from the rest of the biological sample. The biological samples of the second type may then be extracted from the cover lid collector.

Alternatively, the sample carrier engaged in the cover lid collector may be subjected to centrifugation at a first centrifugation speed and subsequently subjected to centrifugation at a second centrifugation speed, whereby first, second and third phases may form, which respectively correspond to biological samples of the first and second types and to the buffer medium, wherein the first phase may be arranged closest to the far end of the cavity and the second phase may be arranged between the first phase and the third phase. The biological samples forming the first phase may then be extracted from the cover lid collector and after that, the biological samples forming the second phase may be extracted from the cover lid collector.

In a preferred embodiment of the invention, the sample carrier comprises a multi-well plate comprising a plurality of wells, wherein when the cover lid collector is engaged with the multi-well plate, the cavity of the cover lid collector is in communication with at least some of the wells of the multi-well plate. In this case, the method may further comprise a step after engaging the cover lid collector with the multi-well plate and before placing the cover lid collector engaged with the multi-well plate in the centrifuge, of turning the cover lid collector engaged with the multi-well plate, such that the multi-well plate is located above the cover lid collector. The cover lid collector engaged with the sample carrier is then placed in the centrifuge in this orientation, i.e. upside down, such that the sample carrier is located over the cover lid collector, opposite to the usual orientation of a sample carrier on a centrifuge.

According to another preferred embodiment of the invention, the sample carrier comprises a hanging-drop plate.

### BRIEF DESCRIPTION OF THE FIGURES

- Fig. 1: shows different views of a cover lid collector according to an embodiment of the invention.
- Fig. 2: shows different views of a cover lid collector according to a different embodiment of the invention.
- Fig. 3: shows different views of the engagement of a cover lid collector according to an embodiment of the invention comprising a sealing element with a sample carrier.
- Fig. 4: shows different views of the a cover lid collector according to an embodiment of the invention comprising a removable separating foil.
- Fig. 5: shows different views of a cover lid collector according to another embodiment of the invention.
- Fig. 6: shows a flow diagram illustrating a method according to an embodiment of the invention.
- Fig. 7: shows different schematic views of the use of a cover lid collector according to the method illustrated in Fig. 6.
- Fig. 8: shows a flow diagram illustrating a method according to a different embodiment of the invention.
- Fig. 9: shows different schematic views of the use of a cover lid collector according to the method illustrated in of Fig. 8.

### DESCRIPTION OF THE PREFERRED EMBODIMENTS

For the purposes of promoting an understanding of the principles of the invention, reference will now be made to a preferred embodiment illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates.

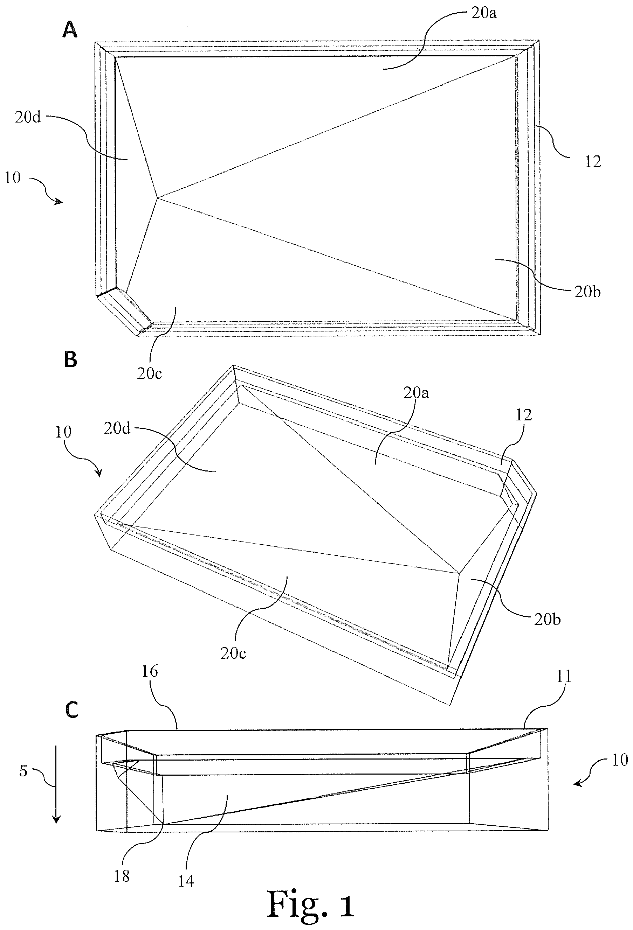

Fig. 1 shows a top view (A), a perspective view (B) and a side view (C) of a cover lid collector 10 according to an embodiment of the invention. The cover lid collector 10 is configured for engaging with a sample carrier for cell cultivation, which is not shown in the figure. The cover lid collector 10 comprises a connecting portion 12 for engaging with the sample carrier so as to close the sample carrier with the cover lid collector 10. For this purpose, the size, dimensions and shape of the cover lid collector 10, and in particular of the connecting portion 12 thereof match those of the sample carrier with which the cover lid collector 10 is to be engaged. In the embodiment shown, the cover lid collector 10 is tailored to a sample carrier that corresponds to a Falcon® 96 Well Clear Flat Bottom TC-Treated Culture Microplate and has a maximum length of 127.63 mm and a maximal width of 85.11 mm, corresponding to the dimensions necessary for being tightly connected to the aforesaid sample carrier. The connecting portion 12 defines a first plane 11.

The cover lid collector 10 further comprises a cavity 14. When the cover lid collector 10 is engaged with the sample carrier, the cavity 14 is in communication with those parts of the sample carrier that are configured for receiving, supporting or containing samples. The cross-section of the cavity 14, defined as the area of a plane parallel to the first plane 11 intersected by the cavity 14, decreases in a direction perpendicular to the first plane 11 that runs between a near end 16 of the cavity 14 and a far end 18 of the cavity 14 away from the sample carrier, that is, away from the first plane 11. This direction is indicated in figure 1C by the vertical arrow 5. In the embodiment shown, the cross-section of the cavity 14 decreases monotonically in the direction indicated by the arrow 5.

In the embodiment shown, the cavity 14 is formed by four planar walls 20a, 20b, 20c, and 20d, which extend between the near end 16 and the far end 18 of the cavity 14. The cross-section of the cavity 14 hence has a polygonal rectangular shape. In the embodiment shown, the cover lid collector 10 comprises an optional indented corner that allows identifying a particular orientation thereof. The planar walls 20c and 20d have a triangular shape, whereas the planar walls 20a and 20b have an irregular quadrilateral shape. The planar walls 20a 20c are opposed to each other, and so are the planar walls 20b and 20d. Further, the planar wall 20a is adjacent to the planar wall 20b and to the planar wall 20d, and so is the planar wall 20c.

The angle of each of the planar walls 20a-20d with the first plane 11 is referred to as the slope angle there. In the embodiment shown, the planar walls 20a and 20c both have a first slope angle of 20°, the planar wall 20b has a second slope angle of 7°, and the planar wall 20d has a third slope angle of 56°. Thus, the cavity 14 displays an asymmetric quasipyramidal shape. The planar walls 20a-20d have a common vertex that forms the far end 18 of the cavity 14. In the embodiment shown, the cover lid collector 10 is made of polystyrene and the planar walls 20a-20d are coated with an antiadhesive coating.

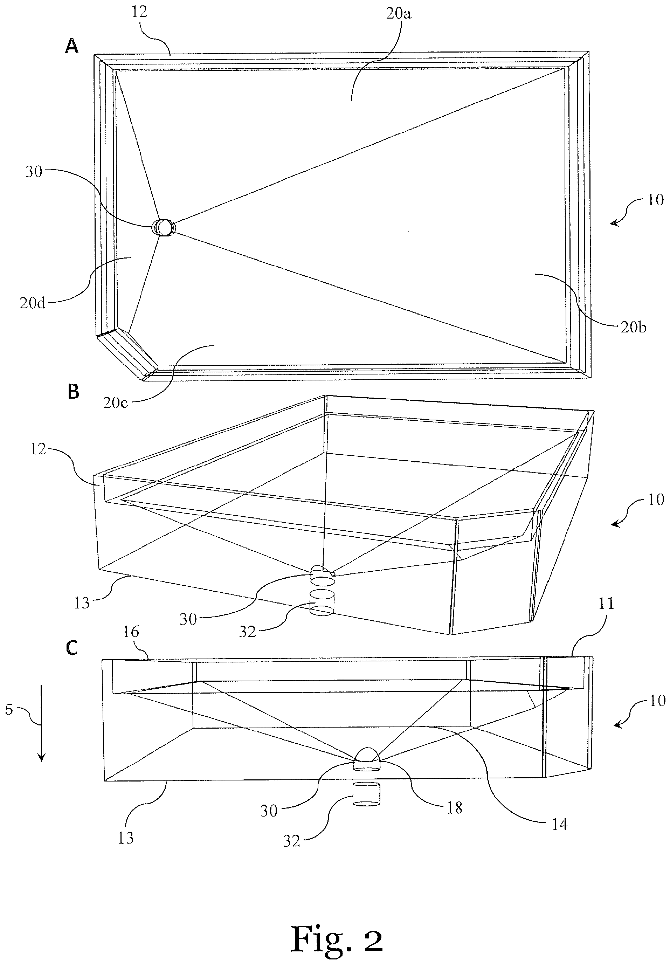

Fig. 2 shows a top view (A), a perspective view (B) and a side view (C) of a cover lid collector 10 according to another embodiment of the invention that comprises all elements described with respect to the embodiment shown in Fig. 1, which are not described here again. In the embodiment shown in Fig. 2, the cover lid collector 10 further comprises a drain opening 30 arranged at the far end 18 of the cavity 14, which far end 18 is formed by the common vertex of the planar walls 20a-20d. The drain opening 30 fluidly connects the interior of the cavity 14 to the exterior of the cover lid collector 10 through the planar surface 13 of the cover lid collector 10, which is parallel to the first plane 11 and separated from it by the cavity 14. The cover lid collector 10 further comprises a sealing cap 32, which is configured for tightly closing the dry opening 30.

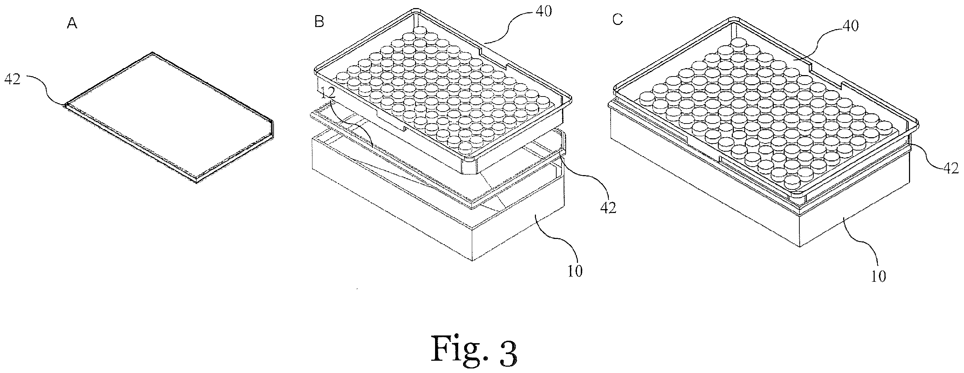

Fig. 3 shows different views of the engagement of a cover lid collector 10 according to an embodiment of the invention with a multi-well plate acting as a sample carrier 40, wherein the cover lid collector 10 further comprises a sealing element 42 that is shown separately in Fig. 3A. The sealing element 42 is configured for being arranged around the cover lid collector 10 and around the sample carrier 40 as illustrated in the exploded view shown in Fig. 3B. The sealing element 42 provides a tight sealed connection between the cover lid collector 10 and the sample carrier 40 when the cover lid collector 10 is engaged with the sample carrier 40 as shown in Fig. 3C.

The sealing element 42 shown in the embodiment of Fig. 3 has a band-like-shape and is made of rubber. The sealing element 42 has a perimeter that exceeds that of the connecting portion 12 of the cover lid collector 10 by 3 mm, whereby a tight connection between the cover lid collector 10 and the sample carrier 10 is possible even when the dimensions of the connecting portion 12 of the cover lid collector 10 do not exactly match those of the sample carrier 40.

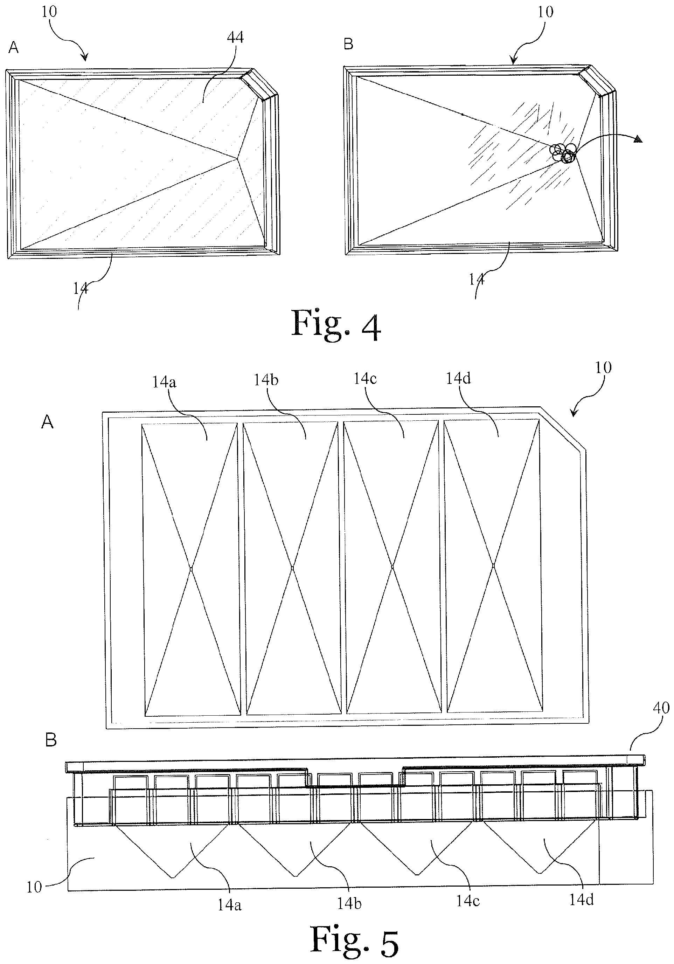

Fig. 4 shows a top view of a cover lid collector 10 according to an embodiment of the invention that comprises a removable separating foil 44 that is configured for covering the near end 16 of the cavity 14 of the cover lid collector 10. The removable separating foil 44 prevents leakage of the samples contained in a sample carrier 40 into the cavity 14 of the cover lid collector 10 when manipulating the cover lid collector 10 and the sample carrier 40 before centrifugation, i.e. in the situation shown in Fig. 4A. The strength of the removable separating foil 44 is chosen such that it may be disrupted or broken by centrifugal forces upon centrifugation, such that once centrifugal forces set in, as shown in Fig. 4B, the removable separating foil 44 no longer blocks access to the cavity 14 for the samples contained in a sample carrier 40 that is engaged with the cover lid collector 10 such that the samples may enter the cavity 14 during centrifugation.

Fig. 5A and 5B respectively show a top view and a side view of a cover lid collector 10 according to an embodiment of the invention that comprises four cavities 14a, 14b, 14c and 14d. When the cover lid collector 10 is engaged with a sample carrier 40, which in the example shown in Fig. 4B corresponds to a multi-well plate, first to fourth parts of the sample carrier 40, which in the embodiment shown respectively correspond to first to fourth groups of wells of the multi-well plate and may respectively contain first to fourth parts of the samples contained in the sample carrier 40, are respectively in communication with the first to fourth cavities 14a to 14d. In the embodiment shown, the first part of the samples are contained in the wells comprised in said first part of the multi-well plate 40, the second part of the samples are contained in the wells comprised in the second part of the multi-well plate 40, and so forth.

When the sample carrier 40 and the cover lid collector 10 are engaged with each other and subjected to centrifugation in order to separate the biological samples from the liquid medium in each of the samples contained in the sample carrier 40, the biological samples comprised in the samples contained in the first part of the sample carrier 40 are collected at the far end of the first cavity 14a, the biological samples comprised in the samples contained in the second part of the sample carrier 40 are collected at the far end of the second cavity 14b, and so forth. In the embodiment shown, the first to fourth cavities 14a to 14d all have a regular pyramidal shape, but other embodiments may comprise cavities of different forms and sizes. In particular, the form and shape of the different cavities may be adapted to the form and shape of a sample carrier or to a distribution pattern of the samples in a sample carrier.

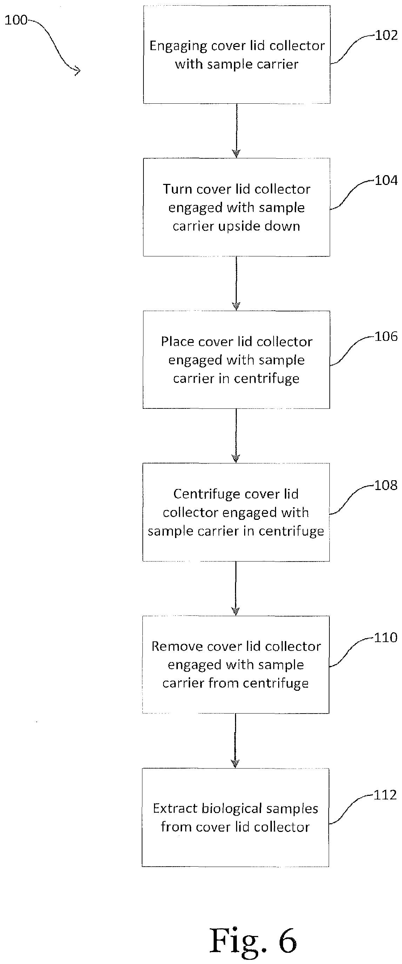

Fig. 6 shows a flow diagram of a method 100 according to an embodiment of the invention of separating and extracting biological samples comprised in a liquid medium contained in a sample carrier 40 corresponding to a multi-well plate 40, wherein the biological samples and the liquid medium constitute samples in the form of a fluid sample that is contained in the wells of the multi-well plate 40. The method 100 foresees the use of a cover lid collector 10 according to any of the embodiments of the present invention described above.

The method 100 comprises a step 102 of engaging the cover lid collector 10 with the multi-well plate 40. As a result of method step 102, the cover lid collector 10 is tightly connected to the multi-well plate 40 by means of the connecting portion 12.

In method step 104, the cover lid collector 10 engaged with the multi-well plate 40 is turned upside down, such that the multi-well plate 40 is now located over the cover lid collector 10. The cover lid collector 10 engaged with the multi-well plate 40 are placed in the centrifuge 50 in this orientation, which is opposite to the usual orientation of a sample carrier in a centrifuge.

The method 100 further comprises a step 106 of placing the cover lid collector 10 engaged with the multi-well plate 40 in a centrifuge 50 such that it may be centrifuged by means of the centrifuge 50

In method step 108, the cover lid collector 10 engaged with the sample carrier 40 is centrifuged using the centrifuge 50. As a consequence of centrifugation, biological samples comprised in the samples contained in the sample carrier 40, i.e. contained in the wells of the multi-well plate 40, are separated from the liquid medium in which they were previously distributed. As explained in detail above, as a consequence of the centrifugation with the cover lid collector 10, the biological samples form a first phase and the liquid medium forms a second phase, wherein the first phase and the second phase are separated from each other, and wherein the first phase is closer to the far end 18 of the cavity 14 of the cover lid collector 10 than the second phase.

In method step 110, the cover lid collector 10 engaged with the sample carrier is removed from the centrifuge 50 after having been subject to centrifugation therein.

The method 100 then continues with step 112, in which biological samples present in the first phase, which is concentrated at the far end 18 of the cavity 14, are extracted from the cover lid collector 10 e.g. by aspirating the biological samples with a laboratory aspirator or by pipetting.

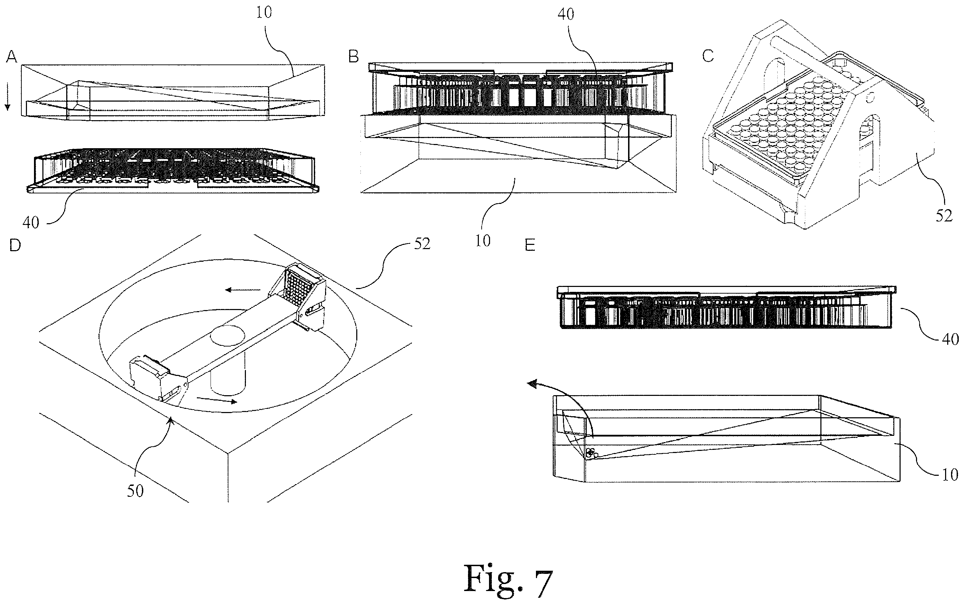

Figs. 7A to 7E show different schematic views of a cover lid collector 10 according to an embodiment of the invention, a sample carrier 40 corresponding to a multi-well plate 40, and a centrifuge 50, which are used according to the method 100 of Fig. 6 of separating and extracting biological samples comprised in a liquid medium, wherein the biological samples and the liquid medium constitute samples in the form of a fluid sample that is contained in the wells of the multi-well plate 40.

Fig. 7A corresponds to snapshot representative of method step 102 in which the cover lid collector 10 is engaged with the multi-well plate 40.

Fig. 7B corresponds to snapshot representative of method step 104 of turning the cover lid collector 10 engaged with the multi-well plate 40 upside down such that they have the orientation shown in the figure in which the multi-well plate 40 is positioned above the cover lid collector 10.

Fig. 7C corresponds to snapshot representative of method step 106 of placing the cover lid collector 10 engaged with the multi-well plate 40 in a centrifuge 50. In the case of the centrifuge 50 shown in Fig. 7, which is an Eppendorf™ 5810R Centrifuge with Rotor Packages supplemented with a swing-bucket rotor with four MTP/Flex carriers, method step 106 comprises placing the cover lid collector 10 engaged with the multi-well plate 40 on a swing-out plate carrier 52 of the centrifuge 50.

Fig. 7D corresponds to snapshot representative of method step 108 of centrifuging the cover lid collector 10 engaged with the multi-well plate 40 in the centrifuge 50 such that centrifugal forces arise in the direction normal to the cover lid collector 10 pointing away from the multi-well plate 40, i.e. pointing towards the far end 18 of the cavity 14, such that biological samples accumulate at the far end 18 of the cavity 14 of the cover lid collector 10.

After centrifugation, the cover lid collector 10 and the multi-well plate 40 are removed from the centrifuge 50 and method step 112 of extracting the biological samples from the cover lid collector 10 is carried out. For this purpose, in the embodiment shown, the cover lid collector 10 and the multi-well plate 40 are disengaged while keeping the orientation shown in Fig. 7B, as shown in Fig. 7E.

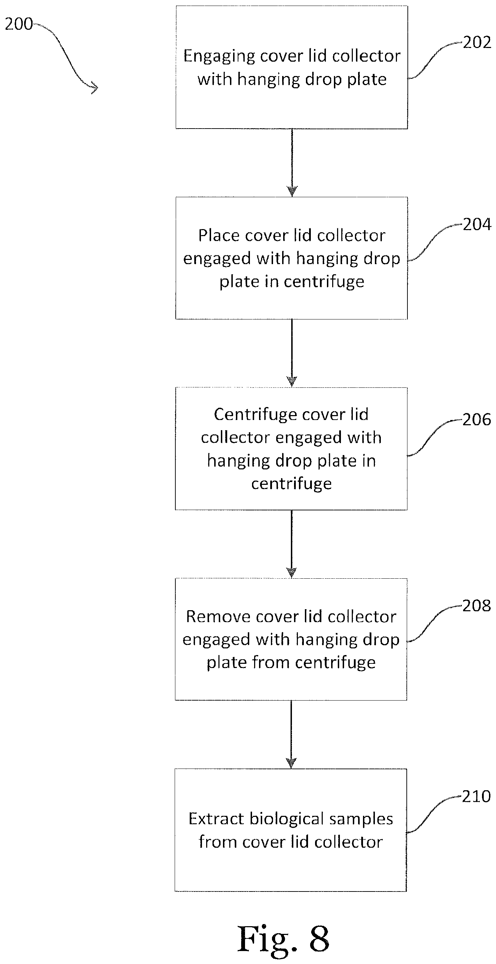

Fig. 8 shows a flow diagram of a method 200 according to another embodiment of the invention of separating and extracting biological samples comprised in a liquid medium contained in a sample carrier 40 corresponding to a hanging drop plate 40, wherein the biological samples and the liquid medium constitute samples in the form of drops that are contained in the hanging drop plate 40. The method 200 foresees the use of a cover lid collector 10 according to any of the embodiments of the present invention described above.

The method 200 comprises a step 202 of engaging the cover lid collector 10 with the hanging drop plate 40. After method step 202, the cover lid collector 10 is tightly connected to the hanging drop plate 40 by means of the connecting portion 12 of the cover lid collector 10.

In method step 204, the cover lid collector 10 engaged with the hanging drop plate 40 is placed in a centrifuge 50 such that it may be centrifuged by means of the centrifuge 50. In the embodiment shown, this comprises placing the cover lid collector 10 engaged with the hanging drop plate 40 on a swing-out plate carrier 52 the centrifuge 50, which also corresponds to an Eppendorf™ 5810R Centrifuge with Rotor Packages supplemented with a swing-bucket rotor with four MTP/Flex carriers.

In method step 206, the cover lid collector 10 engaged with the hanging drop plate 40 is centrifuged using the centrifuge 50. As a result of centrifugation, biological samples comprised in the samples, i.e. in the hanging drops, contained in the hanging drop plate are separated from the liquid medium in which they were previously distributed in a way analogous to method step 108 with reference to Figs. 6 and 7. The biological samples then form a first phase and the liquid medium forms a second phase, wherein the first phase and the second phase are separated from each other, and wherein the first phase is closer to the far end 18 of the cavity 14 of the cover lid collector 10 than the second phase.

In method step 208, the cover lid collector 10 engaged with the hanging drop plate is removed from the centrifuge 50.

In method step 210, the biological samples present in the first phase, which is concentrated at the far end 18 of the cavity 14 of the cover lid collector 10, are extracted from the cover lid collector 10, e.g. by aspirating the biological samples with a laboratory aspirator or by pipetting.

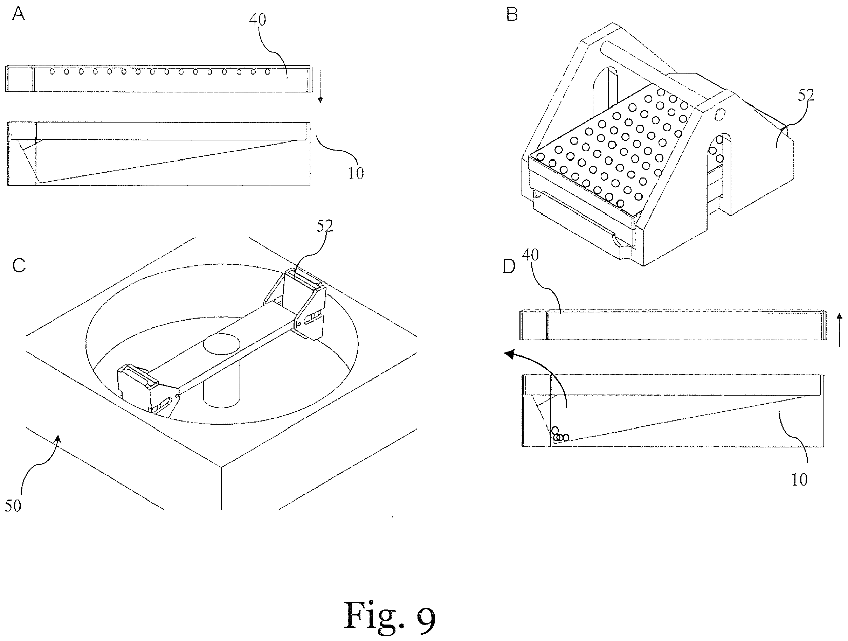

Figs. 9A to 9D show different schematic views of a cover lid collector 10 according to another embodiment of the invention, a sample carrier 40 corresponding to a hanging drop plate 40, and a centrifuge 50, which are used according to the method 200 of Fig. 8 of separating and extracting biological samples comprised in a liquid medium, wherein the biological samples and the liquid medium constitute samples in the form of drops contained in the hanging drop plate 40.

Fig. 9A corresponds to a snapshot representative of method step 202 in which the cover lid collector 10 is engaged with the hanging drop plate 40, wherein the cover lid collector is below the hanging drop plate 40.

Fig. 9B corresponds to snapshot representative of method step 204 of placing the cover lid collector 10 engaged with the hanging drop plate 40 on one of the swing-out plate carriers 52 of a centrifuge 50 in the orientation shown in Fig. 9A.

Fig. 9C corresponds to snapshot representative of method step 206 of centrifuging the cover lid collector 10 engaged with the hanging drop plate 40 in the centrifuge 50 such that centrifugal forces arise in the direction normal to the cover lid collector 10 pointing away from the hanging drop plate 40, i.e. pointing towards the far end 18 of the cavity 14, such that biological samples accumulate at the far end 18 of the cavity 14 of the cover lid collector 10.

After centrifugation, the cover lid collector 10 and the multi-well plate 40 are removed from the centrifuge 50 and method step 210 of extracting the biological samples from the cover lid collector 10 is carried out. For this purpose, in the embodiment shown, the cover lid collector 10 and the hanging drop plate 40 can be disengaged while keeping the orientation shown in Fig. 9B, as shown in Fig. 9D.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

## Claims

1. A cover lid collector (10) for a sample carrier (40) for cell cultivation, wherein the cover lid collector (10) comprises:
a connecting portion (12) for engaging with the sample carrier (40) for closing the same, wherein the connecting portion (12) defines a first plane (11); and
a cavity (14), which when the cover lid collector is engaged with the sample carrier (40), has a cross-section that decreases in a direction perpendicular to the first plane (11) and pointing away from said sample carrier (40) between a near end (16) of the cavity (14) and a far end (18) of the cavity (14), wherein the near end (16) is closer to the sample carrier (40) than the far end (18).

2. The cover lid collector (10) of claim 1, wherein the cross-section of the cavity (14) decreases monotonically in said direction perpendicular to the first plane (11) and pointing away from said sample carrier (40) between a near end (16) of the cavity (14) and a far end (18) of the cavity (14).

3. The cover lid collector (10) of claim 1 or 2, wherein the cavity (14) is formed by a plurality of planar walls (20a-20d) that extend between the near end (16) and the far end (18), such that the cross-section of the cavity (14) has a polygonal shape, wherein at least one of the planar walls (20a-20d) preferably has a polygonal form, preferably a triangular form.

4. The cover lid collector (10) of any of claim 3, wherein one or more of the planar walls (20a-20d) has a slope angle that is different to the slope angle of another one of the planar walls (20a-20d), wherein the slope angle is an angle defined between the surface of the wall and the first plane (11).

5. The cover lid collector (10) of any of claims 2 to 4, wherein the planar walls (20a-20d) have a common vertex (22) that forms the far end (18) of the cavity (14), wherein the cavity (14) is preferably formed by four triangular shaped walls, wherein said four walls are pairwise adjacent and opposed, such that the cavity (14) has a symmetric or asymmetric quasipyramidal shape.

6. The cover lid collector (10) of any of the preceding claims, further comprising a drain opening (30) that fluidly connects the far end (18) of the cavity (14) to the exterior of the cover lid collector (10), wherein the cover lid collector (10) preferably further comprises a sealing cap (32) configured for tightly closing the drain opening (30).

7. The cover lid collector (10) of any of the preceding claims, further comprising a sealing element (42) configured for being arranged around the cover lid collector (10) and around the sample carrier (40) so as to provide a tight sealed connection between the cover lid collector (10) and the sample carrier (40) when the cover lid collector (10) is engaged with the sample carrier (40).

8. The cover lid collector (10) of any of the preceding claims, wherein the cavity (14) and/or at least one of the planar walls (20a-20d) thereof are partly or fully coated with a magnetic material; and/or
wherein the cavity (14) and/or at least one of the sloped walls (20a-20d) thereof are partly or fully coated with an antiadhesive coating; and/or wherein the cavity (14) and/or at least one of the sloped walls (20a-20d) thereof are partly or fully coated with an adhesive coating.

9. The cover lid collector (10) of any of the preceding claims, further comprising a removable separating foil (44) covering the near end (16) of the cavity (14); and/or
wherein the cover lid collector (10) is totally or partially made of a transparent material, such that the interior of the cavity (14) can be seen from the outside of the cover lid collector (10).

10. The cover lid cover lid collector (10) of any of the preceding claims, wherein the cover lid collector (10) comprises one or more cavities (14a-14d).

11. A method of separating and extracting biological samples comprised in a liquid medium, the liquid medium being contained in a sample carrier (40) for cell cultivation, using a cover lid collector (10) that comprises:
a connecting portion (12) for engaging with the sample carrier (40) for closing the same, wherein the connecting portion (12) defines a first plane (11); and
a cavity (14), which when the cover lid collector is engaged with the sample carrier (40), is in fluid communication with at least some of the biological samples, and has a cross-section that decreases in a direction perpendicular to the first plane (11) and pointing away from said sample carrier (40) between a near end (16) of the cavity (14) and a far end (18) of the cavity (14), wherein the near end (16) is closer to the sample carrier (40) than the far end (18),
wherein the method comprises the steps of:
engaging the cover lid collector (10) with the sample carrier (40), such that the cover lid collector (10) is tightly connected to the sample carrier (40);
placing the cover lid collector (10) engaged with the sample carrier (40) in a centrifuge (50), such that the cover lid collector (10) is positioned over the sample carrier (40);
centrifuging the cover lid collector (10) engaged with the sample carrier (40) using the centrifuge (50) such as to subject the liquid medium and the biological samples to a centrifugal force in said direction, such that the biological samples form a first phase and the liquid medium forms a second phase, wherein the first phase and the second phase are separated from each other and wherein the first phase is closer to the far end (18) of the cavity (14) than the second phase; and
extracting the biological sample from the cover lid collector (10).

12. The method of claim 11, wherein the cover lid collector (10) further comprises a drain opening (30) that fluidly connects the far end (18) of the cavity (14) to the exterior of the cover lid collector (10), wherein extracting comprises extracting the second phase through the drain opening (30).

13. The method of claim A or 12, wherein centrifuging comprises centrifuging at different centrifugation speeds.

14. The method of any of claims A to 13, wherein the sample carrier (40) comprises a multi-well plate (40); wherein the method further comprises, after engaging the cover lid collector (10) with the multi-well plate (40) and before placing the cover lid collector (10) engaged with the multi-well plate (40) in the centrifuge (50), a step of turning the cover lid collector (10) engaged with the multi-well plate (40), such that the multi-well plate is located above the cover lid collector (10).

15. The method of any of claims A to 13, wherein the sample carrier (40) comprises a hanging-drop plate (40).
